(19)

Europäisches
Patentamt
European
Patent Office
Office européen
des brevets

(11) **EP 4 450 015 A1**

(12) **EUROPEAN PATENT APPLICATION**
published in accordance with Art. 153(4) EPC

(43) Date of publication:
**23.10.2024 Bulletin 2024/43**

(51) International Patent Classification (IPC):
***A61B 34/30*** *(2016.01)*

(21) Application number: **22906652.7**

(22) Date of filing: **15.12.2022**

(52) Cooperative Patent Classification (CPC):
**A61B 34/00; A61B 34/30**

(86) International application number:
**PCT/CN2022/139328**

(87) International publication number:
**WO 2023/109907 (22.06.2023 Gazette 2023/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.12.2021 CN 202111541632**

(71) Applicant: **Ronovo (Shanghai) Medical Science
and
Technology Ltd.
Shanghai 201102 (CN)**

(72) Inventors:
• **SUN, Xiaowen
  Shanghai 201102 (CN)**
• **JIN, Xin
  Shanghai 201102 (CN)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **DEGREE-OF-FREEDOM REGULATING MECHANISM AND SURGICAL INSTRUMENT**

(57) A degree-of-freedom regulating mechanism (400) and a surgical instrument. The degree-of-freedom regulating mechanism (400) comprises a handle (410), a drive unit group, and a differential mechanism (430). The drive unit group comprises N drive units, wherein N is 3 or 4, and each drive unit is rotatably or linearly movably provided on the handle (410). The differential mechanism (430) is provided on the handle (410). The differential mechanism (430) comprises a first planetary gear train (431, 431') and a corresponding rotating mechanism. Any two gears of the first planetary gear train (431, 431') are configured to independently rotate and are respectively coupled to a drive unit, and the differential mechanism (430) can be switched between a locked state and a released state. When the differential mechanism (430) is in a locked state, a driven wheel of the first planetary gear train (431, 431') and the corresponding rotating mechanism are fixed to each other in the rotation direction, such that the degree of freedom of the degree-of-freedom regulating mechanism (400) is N-1. When the differential mechanism (430) is in a released state, the driven wheel of the first planetary gear train (431, 431') and the corresponding rotating mechanism can rotate independently, such that the degree of freedom of the degree-of-freedom regulating mechanism (400) is N.

Figure 1

**Description**

The present application claims the right of the priority of Chinese patent application 202111541632.5 filed with the CNIPA on December 16, 2021, the contents of which are incorporated herein by reference in their entireties.

TECHNICAL FIELD

The present disclosure relates to the technical field of medical instruments, for example, to a degree-of-freedom regulating mechanism and a surgical instrument.

BACKGROUND

Minimally invasive surgery typically involves making one or more small incisions on the patient's body surface, and inserting the end effectors of various instruments and surgical tools into the patient's body through a trocar at the incision site for diagnosis or treatment. Due to reduced postoperative recovery time and scar size, minimally invasive surgery is usually preferred over open surgery.

Robotic-assisted minimally invasive surgical instruments can provide doctors with a more flexible operating experience by offering additional degrees of freedom to minimally invasive surgery tools in related technologies and simulating the movement of the human wrist. The end effector hinged at the distal end of the surgical tool is typically driven by a pulley-cable mechanism, and moving the drive cable can rotate the end effector to a specified position. However, the pulley-cable mechanism is prone to cable slack and complicated assembly under different operating conditions. To ensure that the end effector and the entire drive mechanism have consistent and predictable performance, it is necessary to overcome transmission and manufacturing defects through optimized design and process.

CONTENT OF THE PRESENT INVENTION

The embodiments of the present disclosure provide a degree-of-freedom regulating mechanism capable of ensuring that a cable remains tensioned under any operating condition.

The embodiments of the present disclosure provide a surgical instrument equipped with the degree-of-freedom regulating mechanism.

According to one aspect of the present disclosure, provided is a degree-of-freedom regulating mechanism, comprising a handle, a drive unit group, and a differential mechanism; the drive unit group comprises N drive units, wherein N is 3 or 4, and each drive unit is rotatably or linearly movably provided on the handle; the differential mechanism is provided on the handle; the differential mechanism comprises a first planetary gear train and a corresponding rotating mechanism; any two gears of the first planetary gear train are configured to independently rotate and are respectively coupled to one of the drive units; the differential mechanism is switchable between a locked state and a released state; wherein when the differential mechanism is in the locked state, a driven wheel of the first planetary gear train and the corresponding rotating mechanism are fixed to each other in the rotation direction, such that the degree of freedom of the degree-of-freedom regulating mechanism is N-1; when the differential mechanism is in the released state, the driven wheel of the first planetary gear train and the corresponding rotating mechanism are able to rotate independently, such that the degree of freedom of the degree-of-freedom regulating mechanism is N.

According to another aspect of the present disclosure, provided is a surgical instrument, comprising an end tool, a cable, and the degree-of-freedom regulating mechanism as proposed in the present disclosure and described in the above embodiments; the drive unit is connected to the end tool via the cable to drive the end tool; wherein the surgical instrument is configured to adjust the degree of freedom of the end tool through the degree-of-freedom regulating mechanism.

BRIEF DESCRIPTION OF THE DRAWINGS

The drawings are merely illustrative of the present disclosure and are not necessarily drawn to scale. In the drawings, the same reference numerals always denote the same or similar components. Specifically:

Figure 1 is a three-dimensional schematic illustration of a degree-of-freedom regulating mechanism combined with a wrist rotating mechanism according to an exemplary embodiment.

Figure 2 is a three-dimensional schematic illustration of the bracket and end tool of the wrist rotating mechanism shown in Figure 1.

Figure 3 is a three-dimensional schematic illustration of the degree-of-freedom regulating mechanism shown in Figure 1.

Figure 4 is a three-dimensional schematic illustration of the differential mechanism shown in Figure 3.

Figure 5 is a sectional view of Figure 4.

Figure 6 is an exploded schematic illustration of the differential mechanism shown in Figure 3.

Figure 7 is a three-dimensional schematic illustration of the planetary gear train in another exemplary embodiment.

Figure 8 is an exploded schematic illustration of the planetary gear train shown in Figure 7.

Figure 9 is a three-dimensional schematic illustration of the planet carrier shown in Figure 6.

Figure 10 is a three-dimensional schematic illustration of the planet carrier in another exemplary embodiment.

Figure 11 is a schematic illustration of the principle of rotation of the differential mechanism shown in Figure 3.

Figure 12 is a three-dimensional assembly schematic illustration of the handle and protective cover of the degree-of-freedom regulating mechanism shown in Figure 1.

The reference numerals are as follows:

| 100, | Bracket; | 423, | Limit stop ring; |
|------|----------|------|-------------------|
| 110, | Sleeve; | 430, | Differential mechanism; |
| 111, | External teeth; | 431, | Planetary gear train; |
| 200, | End tool; | 431', | Planetary gear train; |
| 210, | Subpart; | 4311, | Planet carrier; |
| 310, | First cable; | 43111, | Positioning structure; |
| 320, | Second cable; | 4312, | Sun gear; |
| 400, | Degree-of-freedom regulating mechanism; | 4313, | Ring gear; |
| 410, | Handle; | 4314, | Follower gear; |
| 411, | Adapter; | 4315, | Planetary gear; |
| 420, | Drive shaft; | 432, | Rotatable axle; |
| 421, | Drive gear; | 440, | Steering shaft; |
| 422, | Guide structure; | 441, | Steering gear; |
| | | 450, | Protective cover. |

DETAILED DESCRIPTION OF THE PREFERRED EMBODIMENT

In the following description of various exemplary embodiments of the present disclosure, reference is made to the accompanying drawings, which form a part of the present disclosure, and in which are shown by way of illustration various exemplary structures, systems, and steps that can be implemented to realize multiple aspects of the present disclosure. It should be understood that other specific embodiments of components, structures, exemplary devices, systems, and steps can be utilized, and structural and functional modifications can be made without departing from the scope of the present disclosure. Moreover, although the terms such as "above", "between", and "within" may be used in this specification to describe various exemplary features and elements of the present disclosure, these terms are used herein merely for convenience, for example, according to the example orientations described in the accompanying drawings. Nothing in this specification should be construed as requiring a specific three-dimensional orientation of structures in order to fall within the scope of the present disclosure.

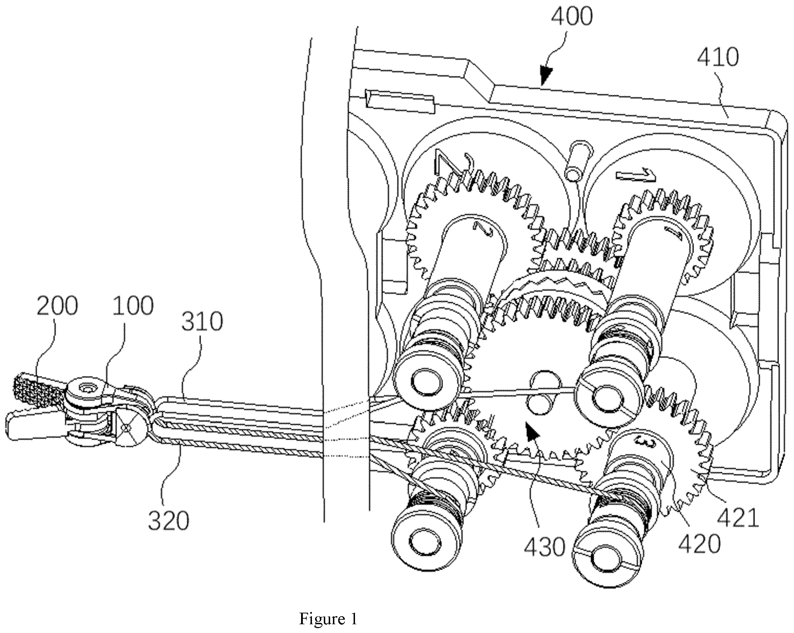

Referring to Figure 1, Figure 1 representatively shows a three-dimensional illustration of a degree-of-freedom regulating mechanism combined with a wrist rotating mechanism proposed in the present disclosure. In this exemplary embodiment, the wrist rotating mechanism proposed in the present disclosure is exemplified using a relevant instrument used in laparoscopic surgery. It should be easily understood by those skilled in the art that various modifications, additions, substitutions, deletions, or other changes can be made to the specific embodiments described below in order to apply the relevant designs of the present disclosure to other types of medical instruments, and these changes still fall within the principles of the degree-of-freedom regulating mechanism proposed in the present disclosure.

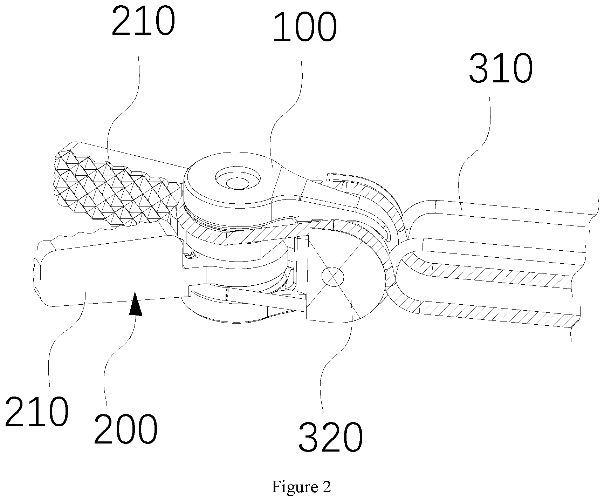

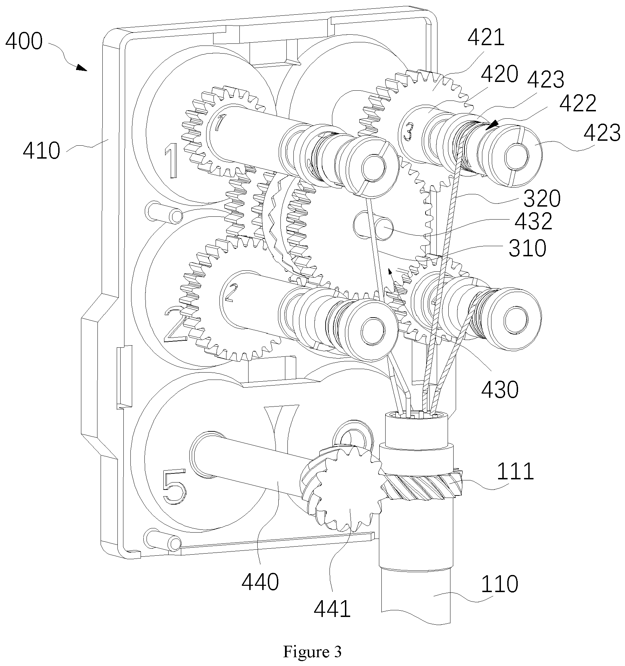

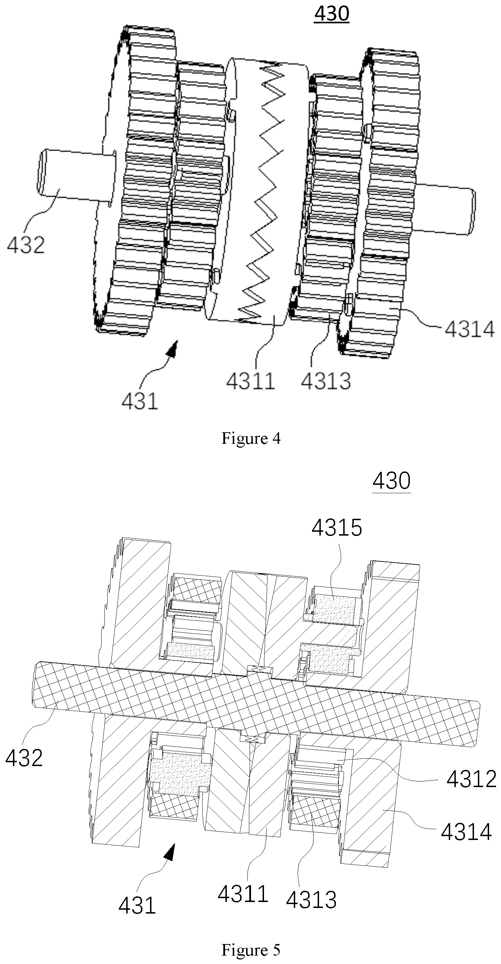

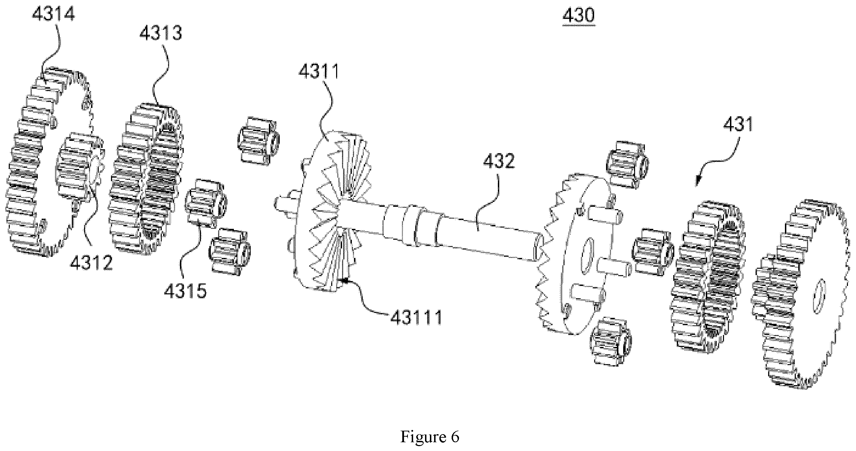

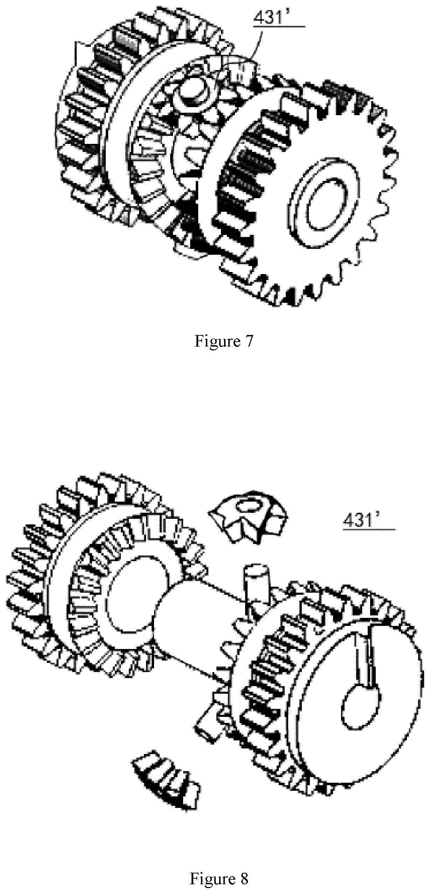

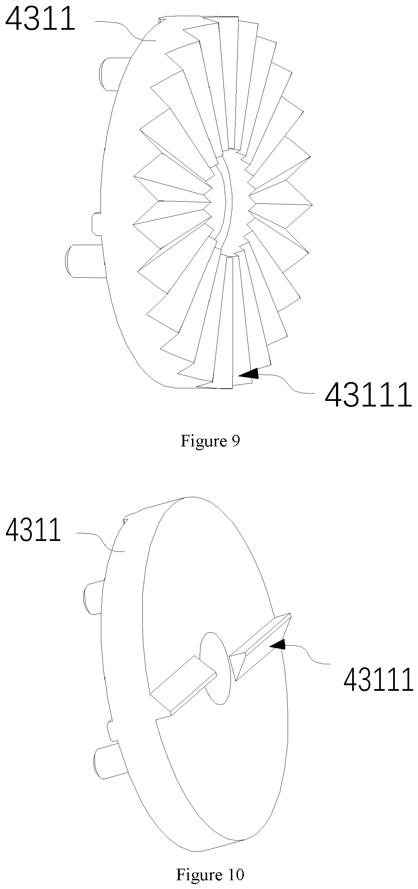

As shown in Figure 1, in this embodiment, the degree-of-freedom regulating mechanism 400 comprises a handle 410, a drive unit group, and a differential mechanism 430. Referring to Figures 2 to 12, Figure 2 shows a three-dimensional schematic illustration of a bracket 100 and an end tool 200. Figure 3 shows a three-dimensional schematic illustration of the degree-of-freedom regulating mechanism 400. Figure 4 shows a three-dimensional schematic illustration of a differential mechanism 430. Figure 5 shows a sectional view of Figure 4. Figure 6 shows an exploded schematic illustration of the differential mechanism 430. Figure 7 shows a three-dimensional schematic illustration of a planetary gear train 431 in another exemplary embodiment. Figure 8 shows an exploded schematic illustration of the planetary gear train 431 shown in Figure 7. Figure 9 shows a three-dimensional schematic illustration of a planet carrier 4311. Figure 10 shows a three-dimensional schematic illustration of the planet carrier 4311 in another exemplary embodiment.

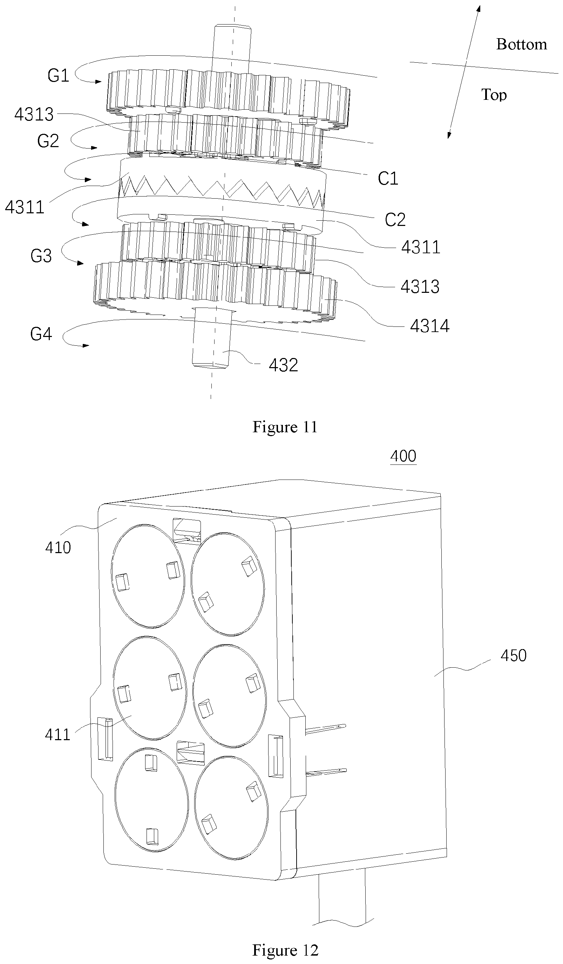

Figure 11 shows a schematic illustration of the principle of rotation of the differential mechanism 430. Figure 12 shows a three-dimensional assembly schematic illustration of a handle 410 and a protective cover 450 of the degree-of-freedom regulating mechanism. In conjunction with the above drawings, a detailed explanation will be provided below regarding the structure, mode of connection, and functional relationship of several main components of the degree-of-freedom regulating mechanism proposed in the present disclosure.

As shown in Figures 1, 3, and 4, in this embodiment, the drive unit group may comprise N drive units, wherein N is 4, and each drive unit is rotatably provided on the handle 410. The differential mechanism 430 is provided on the handle 410. The differential mechanism 430 comprises a planetary gear train and a corresponding rotating mechanism. Any two gears of the planetary gear train can independently rotate and be respectively coupled to one of the drive units, and the differential mechanism 430 is switchable between a locked state and a released state. Accordingly, when the differential mechanism 430 is in the locked state, a driven wheel of the planetary gear train and the corresponding rotating mechanism are fixed to each other in the rotation direction, such that the degree of freedom of the degree-of-freedom regulating mechanism 400 is N-1. When the differential mechanism 430 is in the released state, the driven wheel of the planetary gear train and the corresponding rotating mechanism are able to rotate independently, such that the degree of freedom of the degree-of-freedom regulating mechanism 400 is N. Through the design described above, the present disclosure can ensure that the pulley-cable mechanism remains tensioned under various operating conditions. For example, a robotic-assisted minimally invasive surgical instrument typically has two hinged end effectors, each of which has a rotational degree of freedom. When the mechanism of present disclosure is used to drive these two end effectors, it can be ensured that the cable remains tensioned when the drive unit is coupled to or decoupled from the motor, thus providing consistent and predictable performance of the end effectors and the entire drive mechanism. In some embodiments, the drive unit group may further comprise three drive units, i.e., N may be 3, and each drive unit may also be linearly movably provided on the handle 410, but is not limited thereto.

It should be noted that the degree-of-freedom regulating mechanism 400 of the present disclosure is configured to adjust the free end of a medical instrument, such as a wrist rotating mechanism. For example, it is configured to adjust two or three degrees of freedom at the end of the wrist rotating mechanism, such as the opening and closing, pitching, or yawing of the end tool 200. Therefore, the number of drive units in the degree-of-freedom regulating mechanism 400 of the present disclosure is 3 or 4. The degree-of-freedom regulating mechanism 400 can adjust the degrees of freedom of the drive units according to real-life operating conditions, and adapt to the degrees of freedom of the wrist rotating mechanism described above.

As shown in Figures 1 to 4, the wrist rotating mechanism may comprise a bracket 100 and an end tool 200. The end tool 200 may comprise two subparts 210, each of which is rotatably connected to the distal end of the bracket 100. The bracket 100 and the two subparts 210 are respectively provided with a guide groove. Two cables pass through the guide grooves of the two subparts 210 and the guide groove of the bracket 100, and extend from the proximal end of the bracket 100. The two cables are identified as a first cable 310 and a second cable 320, each having two ends that extend from the proximal end of the bracket 100, resulting in a total of four cable ends, which are respectively connected to the four drive units.

For example, as shown in Figures 1, 3, and 4, in this embodiment, the drive unit may be a drive shaft 420, i.e., the drive unit group comprises four drive shafts 420 that are rotatably provided on the handle 410, and each drive shaft 420 can be individually driven by a motor, which allows the rotational motion of the drive end of the motor to be converted into a linear motion on the cable. The drive shaft 420 is provided with a drive gear 421. Multiple cables respectively correspond to multiple drive shafts 420, with each cable having two ends respectively connected to and wound around two drive shafts 420 of the same pair. Specifically, the rotating mechanism may be another planetary gear train. In other words, the differential mechanism 430 may comprise two planetary gear trains 431, and the two planetary gear trains 431 are coaxially and rotatably provided on the handle 410. The gear of each planetary gear train 431 is bidirectionally driven by the drive shaft 420 to which it is coupled. A locking mechanism is provided between the driven wheels of the two planetary gear trains 431, and the locking mechanism is configured to restrict the relative rotation between the driven wheels of the two planetary gear trains.

For example, as shown in Figures 1, 3, and 4, in this embodiment, the ring gear 4313 of each planetary gear train 431 has external teeth 111. Specifically, multiple pairs of drive shafts 420 respectively correspond to two planetary gear trains 431. The drive gears 421 of the two drive shafts 420 of the same pair are respectively meshed with the external teeth 111 of the ring gear 4313 and the sun gear 4312 of one planetary gear train 431, and the multiple drive gears 421 do not interfere with each other. In some embodiments, the connection between the planetary gear train and the drive shaft, i.e., between the differential mechanism and the drive unit group, may also be achieved by other structures in the form of a transmission mechanism. For example, the transmission mechanism may also include a flexible pulley-cable transmission mechanism or a rigid transmission mechanism, but is not limited thereto.

Through the design described above, when the present disclosure is applied to the wrist rotating mechanism, it enables the adjustment of multiple degrees of freedom of the end tool 200 through multiple cables driven by the drive unit, ensuring that the cables remain tensioned under any operating conditions. On such basis, the degree-of-freedom regulating mechanism 400, equipped with the differential mechanism 430, can compensate for cable slack, ensuring that the cables remain tensioned throughout their entire life cycle, which enhances the precision and reliability of the operation of the instrument. Additionally, the degree-of-freedom regulating mechanism 400 can convert the drive of the motor into the linear motion of the cable. Moreover, through the differential mechanism 430, it can eliminate an extra degree of freedom prior to the loading of the instrument, preventing multiple cables from loosening. This design simplifies the structural complexity and reduces the number of components compared to solutions in related art.

Furthermore, based on the design concept of the degree-of-freedom regulating mechanism 400 of the present disclosure, the three degrees of freedom at the end of the wrist rotating mechanism (opening and closing, pitching, and yawing of the end tool 200) are required to be jointly driven by the four ends of two cables. Therefore, it is required to keep the total length of the four segments of the two cables constant during the driving process (excluding any redundant winding around the drive shaft 420 within the handle 410). To ensure that the cable is not loosened when the wrist rotating mechanism is not loaded to the drive unit, and that it can function normally after loading, the degree-of-freedom regulating mechanism 400 still maintains the function of keeping the total length of the cable constant without the control of the drive unit.

For example, as shown in Figures 1 to 3, in this embodiment, the four ends of the two cables are respectively connected to and wound around the four drive shafts 420. The drive gears 421 of the two drive shafts 420 (for example, the No. 1 and No. 2 drive shafts 420 as illustrated) connected to the two ends of the first cable 310 are respectively meshed with one planetary gear train 431, and the drive gears 421 of the two drive shafts 420 (for example, the No. 3 and No. 4 drive shafts 420 as illustrated) connected to the two ends of the second cable 320 are respectively meshed with another planetary gear train 431.

Additionally, as shown in Figures 1 and 3, labels "1", "2", and "3" are marked on or adjacent to the drive shafts 420 in the accompanying drawings (the label "4" on or adjacent to another drive shaft 420 is not shown due to being obscured). For this purpose, these labels can be set using integrally molded protrusions or recesses, or using printing or other methods. Furthermore, other methods can also be used to distinguish multiple drive shafts 420, such as applying different patterns or colors, which are not limited to this embodiment. Additionally, in some embodiments, these labels may not be necessary, *i.e.*, these labels in the drawings can be understood as schematic labels, and are intended solely to facilitate understanding and explanation.

For example, before the drive unit is loaded, the differential mechanism 430 can ensure that the length of the cable remains constant. After the drive unit is loaded, the drive unit itself can ensure that the length of the cable remains constant, at which point the differential mechanism 430 can either follow passively or remain inactive. When slack occurs in the cable, the cable can be tightened through the drive unit. On such basis, the differential mechanism 430 employs a unidirectional engagement design, which ensures that the differential mechanism 430 does not affect the function of the drive unit to tighten the cable during use. Accordingly, the present disclosure can maintain a constant length of the cable through the differential mechanism 430, *i.e.*, constraining the extra degree of freedom in the N+1 drive scheme through the differential mechanism 430.

For example, as shown in Figures 3 to 6, in this embodiment, a rotatable axle 432 may be fixedly connected to the handle 410, and the two planetary gear trains 431 are each rotatably provided on the rotatable axle 432. For any planetary gear train 431, the rotatable axle 432 can be inserted through the central hole of the planet carrier 4311 and the sun gear 4312. The sun gear 4312 is fixedly connected to a coaxial follower gear 4314. The ring gear 4313 is coaxially arranged at a distance from the sun gear 4312. The planet carrier 4311 is provided with multiple planetary gears 4315 between the sun gear 4312 and the ring gear 4313. Each planetary gear 4315 meshes with the inner teeth of the ring gear 4313 and the sun gear 4312. On such basis, the drive gears 421 of the two drive shafts 420 of the same pair are respectively meshed with the external teeth 111 of the ring gear 4313 and the follower gear 4314 of a planetary gear train 431.

For example, in this embodiment, the sun gear 4312 and the follower gear 4314 may be an integral structure for one planetary gear train 431. Additionally, in some embodiments, the sun gear 4312 may also be fixedly connected to the follower gear 4314.

For example, as shown in Figure 6, in this embodiment, the number of planetary gears 4315 may be three for one planetary gear train 431. Additionally, in some embodiments, the number of planetary gears 4315 may also be one, two, four, or more than four.

As described above, each of the follower gears 4314 (including the sun gear 4312) and the ring gear 4313 can be driven to rotate by the corresponding drive gear 421 on the drive shaft 420, thereby driving the planet carrier 4311 to rotate. The rotational speed of the planet carrier 4311 can be adjusted by adjusting the gear ratio of the gear train. Specifically, the two planetary gear trains 431 are arranged axially relative to each other. Since the two planet carriers 4311 have a relative positioning that prevents them from rotating relative to each other, it is evident that each planetary gear train 431 has two degrees of freedom. However, the relative positioning between the two planet carriers 4311 restricts one rotational degree of freedom, thus reducing the four degrees of freedom of the entire wrist rotating mechanism to three.

As shown in Figure 11, taking the pitching of the wrist of the bracket 100 as an example, if the pitching joint rotates in the clockwise direction as illustrated, and the end tool 200 remains stationary, then the two segments of the second cable 320 are tightened while the two segments of the first cable 310 are released. According to the winding direction of the two cables around the drive shaft 420 in Figure 11, all four drive shafts 420 rotate in the clockwise direction shown in Figure 11. In some embodiments, the winding direction of the four segments of cables (*i.e.*, the four ends of the two cables) around the corresponding drive shafts 420 can also be opposite to what is shown in Figure 11. The arrangement is not limited to the exact configuration illustrated, and the winding direction between the cables need not to be consistent, as long as the cables can be tightened or released by the drive ends as needed.

As shown in Figure 11, the drive gears 421 on the four drive shafts 420 correspondingly drive the follower gears 4314 (*i.e.*, the sun gears 4312) and the ring gears 4313 of the differential mechanism 430 to rotate in the counterclockwise direction as illustrated. In the figure, the two planet carriers 4311 will rotate in the counterclockwise direction as illustrated. It is certain that the rotational states of the multiple components of the two planetary gear trains 431 shown in Figure 11 are purely exemplary. In practice, the differential mechanism 430 can also be understood as a differential, where the adjustment of the gear ratio can make the rotational speed of each planet carrier 4311 equal to the difference between the rotational speeds of the corresponding pair of drive shafts 420.

For example, suppose that the rotational speed of the No. 1 drive shaft 420 is $n_1$, the rotational speed of the No. 2 drive shaft 420 is $n_2$, the rotational speed of the No. 3 drive shaft 420 is $n_3$, the rotational speed of the No. 4 drive shaft 420 is $n_4$, the rotational speed of the planet carrier 4311 positioned above in the figure is nci, and the rotational speed of the planet carrier 4311 positioned below in the figure is $n_{C2}$. The gear ratios of the drive shaft 420 to the corresponding spur gear (follower gear 4314 or ring gear 4313) of the differential mechanism 430 are $GR_1$, $GR_2$, $GR_3$, and $GR_4$; $GR_1$ corresponds to the sun gear tooth count zsi, $GR_2$ corresponds to the internal gear tooth count $z_{I1}$, $GR_4$ corresponds to the sun gear tooth count $z_{S2}$, and $GR_3$ corresponds to the internal gear tooth count $z_{I2}$. When the following relationships are satisfied, the differential mechanism 430 can eliminate one degree of freedom in the wrist rotating mechanism, ensuring that the cables remain tensioned in any state.

$$z_{S1} * n_1 * GR_1 + z_{I1} * n_2 * GR_2 = (z_{S1} + z_{I1}) * n_{C1}$$

$$z_{S2} * n_4 * GR_4 + z_{I2} * n_3 * GR_3 = (z_{S2} + z_{I2}) * n_{C2}$$

$$n_{C1} = n_{C2}$$

For example, as shown in Figures 4 to 6, in this embodiment, multiple drive shafts 420 may be arranged circumferentially around the rotatable axle 432. On such basis, the outer diameter of the follower gear 4314 may be larger than the outer diameter of the ring gear 4313, and the outer diameter of the drive gear 421 meshed with the follower gear 4314 may be smaller than the outer diameter of the drive gear 421 meshed with the external teeth 111 of the ring gear 4313.

For example, based on the design of multiple drive shafts 420 arranged circumferentially around the rotatable axle 432, in this embodiment, the spacing between the multiple drive shafts 420 and the rotatable axle 432 may be equal.

For example, as shown in Figures 3 to 6, based on the design of multiple drive shafts 420 arranged circumferentially around the rotatable axle 432, in this embodiment, the heights at which the drive gears 421 of the multiple drive shafts 420 are arranged along the axial direction of the drive shaft 420 are not the same. Moreover, the heights at which the multiple drive gears 421 are arranged may respectively correspond to the arrangement heights of the follower gears 4314 or ring gears 4313 with which they are meshed. Accordingly, the present disclosure can ensure that the multiple drive gears 421 do not interfere with each other spatially.

For example, as shown in Figures 5 and 6, in this embodiment, the axial direction of the planetary gear 4315 may be parallel to the axial direction of the rotatable axle 432. For example, for one planetary gear train 431, one end of the rotatable axle 432 passes through the central hole of the planet carrier 4311, the sun gear 4312 is provided on this end of the rotatable axle 432, and the follower gear 4314 is located on the side of the sun gear 4312 opposite to the planet carrier 4311. The ring gear 4313 is arranged circumferentially around the sun gear 4312 and is generally located between the planet carrier 4311 and the follower gear 4314. The inner ring of the ring gear 4313 is provided with internal teeth. The planetary gear 4315 is provided on the side of the planet carrier 4311 facing the follower gear 4314 and is located in the annular space formed between the sun gear 4312 and the ring gear 4313. The planetary gear 4315 is meshed with both the external teeth 111 of the sun gear 4312 and the internal teeth of the ring gear 4313.

Additionally, the planetary gear train can also adopt other structural forms of gear set designs. For example, as shown in Figures 7 and 8, in some embodiments, the axial direction of the planetary gears of the planetary gear train 431' can be perpendicular to the axial direction of the rotatable axle, and can also be used as a differential mechanism in the present disclosure.

For example, as shown in Figures 6 and 9, in this embodiment, positioning structures 43111 may be respectively provided on opposing sides of the planet carriers 4311 of the two planetary gear trains 431. Accordingly, the two planet carriers 4311 are in contact with each other on their respective opposing sides, and the positioning structures 43111 of the two planet carriers 4311 are snap-fit to each other, so that the two planet carriers 4311 are fixed relative to each other in the rotation direction with no relative rotation.

For example, as shown in Figures 6 and 9, in this embodiment, the positioning structures 43111 of the two planet carriers 4311 may respectively be claw-shaped structures. The claws of the claw-shaped structures of the two planet carriers 4311 are snap-fit to each other to achieve the positioning of the two planet carriers 4311.

Additionally, the planet carrier 4311 may also adopt other structural forms of positioning structures 43111. For example, as shown in Figure 10, in some embodiments, taking two planetary gear trains 431 as an example, the positioning structure 43111 of one planet carrier 4311 may be a key (such as a unidirectional or bidirectional key), and the positioning structure 43111 of the other planet carrier 4311 may be a keyway. The key is snap-fit to the keyway to achieve the positioning of the two planet carriers 4311.

Additionally, in some embodiments, a coaxially arranged positioning hole is provided between the driven wheels of the two planetary gear trains. The positioning hole can be locked by a pin that moves axially, thereby achieving relative fixation of the two planetary gear trains in the rotation direction.

For example, as shown in Figure 3, in this embodiment, the drive shaft 420 is defined as having a first end and a second end, with the first end rotatably connected to the handle 410. On such basis, the second end of the drive shaft 420 may be provided with a guide structure 422, with the end of the cable connected to and wound around the guide structure 422, and the drive gear 421 is located between the guide structure 422 and the second end.

For example, based on the design of the second end of the drive shaft 420 provided with a guide mechanism, in this embodiment, the guide structure 422 may comprise a guide arc surface or a guide column.

For example, as shown in Figure 3, based on the design of the second end of the drive shaft 420 provided with a guide mechanism, in this embodiment, the guide structure 422 may be provided with a limit stop ring 423 at each axial end of the drive shaft 420. The limit stop rings 423 can restrict the winding position of the cable on the guide structure 422.

For example, as shown in Figure 3, in this embodiment, a sleeve 110 may be connected to the proximal end of the bracket 100. The cable extending from the proximal end of the bracket 100 can pass through the sleeve 110, extend from the proximal end of the sleeve 110, and finally be connected to and wound around the drive shaft 420. On such basis, the proximal end of the sleeve 110 may be provided with external teeth 111. The degree-of-freedom regulating mechanism 400 may further comprise a steering shaft 440. The steering shaft 440 is rotatably provided on the handle 410 and is driven by a drive unit. The steering shaft 440 is provided with a steering gear 441, and the steering gear 441 is meshed with the external teeth 111 of the sleeve 110. Through the design described above, the present disclosure can drive the steering shaft 440 via the drive unit to drive the sleeve 110 to rotate about its own axis, thereby driving the bracket 100 and the end tool 200 at the distal end of the sleeve 110 to rotate.

For example, as shown in Figure 12, in this embodiment, the handle 410 may be provided with multiple adapters 411, and these adapters 411 are rotatably provided on the handle 410. On such basis, multiple drive shafts 420 can be respectively fixed to the multiple adapters 411, so that multiple drive units are respectively connected through the multiple adapters 411. The drive units connect and drive the adapters 411 to rotate, thereby driving the corresponding drive shafts 420 to rotate synchronously. Additionally, taking the degree-of-freedom regulating mechanism 400 in this embodiment, which comprises four drive shafts 420, as an example, the number of adapters 411 provided on the handle 410 can be greater than the number of drive shafts 420. The additional adapters 411 can be used for selectively providing other components (such as the steering shaft 440) or left for further use.

For example, as shown in Figure 12, in this embodiment, the degree-of-freedom regulating mechanism 400 may further comprise a protective cover 450. For example, the protective cover 450 is detachably provided on the handle 410, for example, but not limited to, using a snap-fit structure. When the protective cover 450 is connected to the handle 410, the two can together form a cavity, which can accommodate other components of the degree-of-freedom regulating mechanism 400, such as the drive shaft 420 and the differential mechanism 430. Additionally, the drive unit may be provided within the cavity or outside the cavity, such as on the side of the handle 410 opposite to the protective cover 450.

Based on the above detailed description of one embodiment of the present disclosure, a brief description of another embodiment of the present disclosure is given below. In this embodiment, the drive unit group comprises three drive units, *i.e.*, N is 3. In this embodiment, the degree-of-freedom regulating mechanism proposed in the present disclosure uses a design that is substantially similar to the embodiment described above. The difference is that when N is 3, the rotating mechanism of the differential mechanism is a rotatable axle, *i.e.,* the differential mechanism comprises a planetary gear train and a rotatable axle.

In some embodiments, when the drive unit group comprises three drive units, *i.e.,* N is 3, the rotating mechanism may also be a rotatable axle. The rotatable axle and the planetary gear train are coaxially and rotatably provided on the handle. The gear of the planetary gear train is bidirectionally driven by the drive unit to which it is coupled. The rotatable axle is coupled with one drive unit, and the rotatable axle is bidirectionally driven by the drive unit to which it is coupled. A locking mechanism is provided between the driven wheel of the planetary gear train and the rotatable axle, and the locking mechanism is configured to restrict the relative rotation between the driven wheel of the planetary gear train and the rotatable axle.

In some embodiments, when the drive unit group comprises three drive units, *i.e.,* N is 3, a positioning structure is provided between the driven wheel of the planetary gear train and the rotatable axle. The positioning structure can achieve relative fixation of the planetary gear train and the rotatable axle in the rotation direction through snap-fit.

In some embodiments, when the drive unit group comprises three drive units, *i.e.,* N is 3, the positioning structure may be a claw-shaped structure respectively provided on the driven wheel of the planetary gear train and the rotatable axle, with the claws of the two claw-shaped structures snap-fit to each other. Alternatively, the positioning structure may also be a key provided on one of the driven wheel of the planetary gear train and the rotatable axle, and a keyway provided on the other of the two. The key is snap-fit to the keyway.

In some embodiments, when the drive unit group comprises three drive units, *i.e.,* N is 3, a coaxially arranged positioning hole may be provided between the driven wheel of the planetary gear train and the rotatable axle. The positioning hole can be locked by a pin that moves axially, thereby achieving relative fixation of the planetary gear train and the rotatable axle in the rotation direction.

It should be noted that the degree-of-freedom regulating mechanisms shown in the accompanying drawings and described in this specification are merely a few examples of the many types of degree-of-freedom regulating mechanisms that can utilize the principles of the present disclosure. It should be clearly understood that the principles of the present disclosure are in no way limited to any details or components of the degree-of-freedom regulating mechanisms shown in the accompanying drawings or described in this specification.

Based on the above detailed description of several exemplary embodiments of the degree-of-freedom regulating mechanism proposed in the present disclosure, exemplary embodiments of the surgical instrument proposed in the present disclosure will be described below.

In this embodiment, the surgical instrument proposed in the present disclosure comprises an end tool, a cable, and the degree-of-freedom regulating mechanism proposed in the present disclosure and described in detail in the above embodiments. Specifically, the drive unit of the degree-of-freedom regulating mechanism is connected to the end tool via the cable to drive the end tool. Since the number of drive units allows for one more degree of freedom than that of the end tool, when the end tool is not loaded to the drive unit, the degree-of-freedom regulating mechanism can eliminate the extra degree of freedom while ensuring that the cable remains tensioned; when the end tool is loaded to the drive unit, the degree-of-freedom regulating mechanism can release the extra degree of freedom. In the state where the extra degree of freedom is released, the cable tension is ensured by the coupled movement of the drive unit. If the choice is made to continue restricting the extra degree of freedom, the degree-of-freedom regulating mechanism can prevent cable slack. Accordingly, the surgical instrument proposed in the present disclosure can adapt to the degree of freedom of the end tool through the degree-of-freedom regulating mechanism, ensuring that the cable remains in a tensioned state under any operating conditions.

It should be noted that the surgical instruments shown in the accompanying drawings and described in this specification are merely a few examples of the many types of surgical instruments that can utilize the principles of the present disclosure. It should be clearly understood that the principles of the present disclosure are in no way limited to any details or components of the surgical instruments shown in the accompanying drawings or described in this specification.

Based on the above detailed description of one exemplary embodiment of the surgical instrument proposed in the present disclosure, exemplary embodiments of the surgical robot proposed in the present disclosure will be described below.

In this embodiment, the surgical robot proposed in the present disclosure comprises the surgical instrument proposed in the present disclosure and described in detail in the above embodiment.

It should be noted that the surgical robots shown in the accompanying drawings and described in this specification are merely a few examples of the many types of surgical robots that can utilize the principles of the present disclosure. It should be clearly understood that the principles of the present disclosure are in no way limited to any details or components of the surgical robots shown in the accompanying drawings or described in this specification.

The exemplary embodiments of the degree-of-freedom regulating mechanism, surgical instrument, and surgical robot proposed in the present disclosure have been described and/or illustrated in detail above. However, the embodiments of the present disclosure are not limited to the specific embodiments described herein. On the contrary, the components and/or steps of each embodiment can be used independently and separately from the other components and/or steps described herein. Each component and/or step of one embodiment can also be used in combination with other components and/or steps of other embodiments. When introducing the elements/components/etc. described and/or illustrated herein, the terms "a", "an", and "the" are used to indicate that there are one or more elements/components/etc.

The terms "comprise", "include", and "have" are used to indicate open-ended inclusion and mean that there may be additional elements/components/etc. in addition to the listed elements/components/*etc*. Moreover, the terms "first" and "second" in the claims and specification are used merely as labels and do not impose numerical limitations on their objects.

**Claims**

1. A degree-of-freedom regulating mechanism, comprising a handle, a drive unit group, and a differential mechanism;

   the drive unit group comprises N drive units, wherein N is 3 or 4, and each drive unit is rotatably or linearly movably provided on the handle;
   the differential mechanism is provided on the handle; the differential mechanism comprises a first planetary gear train and a corresponding rotating mechanism; any two gears of the first planetary gear train are configured to independently rotate and are respectively coupled to one of the drive units; the differential mechanism is switchable between a locked state and a released state;
   wherein when the differential mechanism is in the locked state, a driven wheel of the first planetary gear train and the corresponding rotating mechanism are fixed to each other in the rotation direction, such that the degree of freedom of the degree-of-freedom regulating mechanism is N-1; when the differential mechanism is in the released state, the driven wheel of the first planetary gear train and the corresponding rotating mechanism are able to rotate independently, such that the degree of freedom of the degree-of-freedom regulating mechanism is N.

2. The degree-of-freedom regulating mechanism according to claim 1, wherein N is 3;

   the rotating mechanism is a rotatable axle, and the rotatable axle and the first planetary gear train are coaxially and rotatably provided on the handle;
   the gear of the first planetary gear train is bidirectionally driven by the drive unit coupled with the planetary gear;
   the rotatable axle is coupled with one drive unit; the rotatable axle is bidirectionally driven by the drive unit coupled with the rotatable axle;
   a locking mechanism is provided between the driven wheel of the first planetary gear train and the rotatable axle, and the locking mechanism is configured to restrict the relative rotation between the driven wheel of the first planetary gear train and the rotatable axle.

3. The degree-of-freedom regulating mechanism according to claim 2, wherein
   a positioning structure is provided between the driven wheel of the first planetary gear train and the rotatable axle, and the positioning structure is configured to achieve relative fixation of the first planetary gear train and the rotatable axle in the rotation direction through snap-fit.

4. The degree-of-freedom regulating mechanism according to claim 3, wherein

   the positioning structure is a claw-shaped structure respectively provided on the driven wheel of the first planetary gear train and the rotatable axle, with claws of the two claw-shaped structures snap-fit to each other; or
   the positioning structure is a key provided on the driven wheel of the first planetary gear train and a keyway provided on the rotatable axle, or the positioning structure is a keyway provided on the driven wheel of the first planetary gear train and a key provided on the rotatable axle, wherein the key is snap-fit to the keyway.

5. The degree-of-freedom regulating mechanism according to claim 2, wherein a coaxially arranged positioning hole is provided between the driven wheel of the first planetary gear train and the rotatable axle, and the positioning hole is configured to be locked by a pin that moves axially, thereby achieving relative fixation of the first planetary gear train and the rotatable axle in the rotation direction.

6. The degree-of-freedom regulating mechanism according to claim 1, wherein N is 4;

   the rotating mechanism is a second planetary gear train, and the first planetary gear train and the second planetary gear train are coaxially and rotatably provided on the handle;
   the gear of the first planetary gear train is bidirectionally driven by the drive unit coupled with the first planetary gear train, and the gear of the second planetary gear train is bidirectionally driven by the drive unit coupled with the second planetary gear train;

a locking mechanism is provided between the driven wheel of the first planetary gear train and the driven wheel of the second planetary gear train, and the locking mechanism is configured to restrict the relative rotation between the driven wheel of the first planetary gear train and the driven wheel of the second planetary gear train.

7. The degree-of-freedom regulating mechanism according to claim 6, wherein a positioning structure is provided between the driven wheels of the two planetary gear trains, and the positioning structure is configured to achieve relative fixation of the two planetary gear trains in the rotation direction through snap-fit.

8. The degree-of-freedom regulating mechanism according to claim 7, wherein the positioning structure is a claw-shaped structure respectively provided on the driven wheels of the two planetary gear trains, with claws of the two claw-shaped structures snap-fit to each other; or
the positioning structure is a key and a keyway respectively provided on the driven wheels of the two planetary gear trains, wherein the key is snap-fit to the keyway.

9. The degree-of-freedom regulating mechanism according to claim 6, wherein
a coaxially arranged positioning hole is provided between the driven wheels of the two planetary gear trains, and the positioning hole is configured to be locked by a pin that moves axially, thereby achieving relative fixation of the two planetary gear trains in the rotation direction.

10. The degree-of-freedom regulating mechanism according to claim 1, wherein
a transmission mechanism is connected between the differential mechanism and the drive unit group, and the transmission mechanism comprises a flexible pulley-cable transmission mechanism or a rigid transmission mechanism.

11. The degree-of-freedom regulating mechanism according to claim 1, wherein the handle is detachably provided with a protective cover, and the protective cover and the handle together form a cavity for accommodating the drive unit and the differential mechanism.

12. A surgical instrument, comprising an end tool, a cable, and the degree-of-freedom regulating mechanism according to any one of claims 1 to 11;

the drive unit is connected to the end tool via the cable to drive the end tool;
wherein the surgical instrument is configured to adjust the degree of freedom of the end tool through the degree-of-freedom regulating mechanism.

Figure 1

210    100    310

210    200    320

Figure 2

Figure 3

430

Figure 4

430

4315

Figure 5

Figure 6

EP 4 450 015 A1

Figure 7

Figure 8

4311

43111

Figure 9

4311

43111

Figure 10

G1

4313

G2

4311

G3

G4

Bottom

Top

C1

C2

4311

4313

4314

432

Figure 11

400

410

411

450

Figure 12

## INTERNATIONAL SEARCH REPORT

| International application No. |
|---|
| **PCT/CN2022/139328** |

**A. CLASSIFICATION OF SUBJECT MATTER**

A61B34/30(2016.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNKI, CNPAT, WPI, EPODOC: 瑞龙诺赋, 孙晓文, 金鑫, 诺曼·亚历克斯, 手术, 外科, 微创, 差速, 行星齿, surgical, effector, robot, drive, shaft, epicyclic gear, planet gear, wheel

**C. DOCUMENTS CONSIDERED TO BE RELEVANT**

| Category* | Citation of document, with indication, where appropriate, of the relevant passages | Relevant to claim No. |
|---|---|---|
| A | CN 113197671 A (RUILONG NUOFU (SHANGHAI) MEDICAL TECHNOLOGY CO., LTD.) 03 August 2021 (2021-08-03)<br>description, paragraphs [0058]-[0066] | 1-12 |
| A | CN 104602619 A (ETHICON ENDO SURGERY INC.) 06 May 2015 (2015-05-06)<br>entire document | 1-12 |
| A | CN 113272104 A (COVIDIEN LP) 17 August 2021 (2021-08-17)<br>entire document | 1-12 |
| A | US 2020275928 A1 (ETHICON L.L.C.) 03 September 2020 (2020-09-03)<br>entire document | 1-12 |
| A | WO 2016026519 A1 (POLESTAR PERFORMANCE AB.) 25 February 2016 (2016-02-25)<br>entire document | 1-12 |
| A | CN 112087983 A (COVIDIEN LP) 15 December 2020 (2020-12-15)<br>entire document | 1-12 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 February 2023** | **03 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
**Information on patent family members**

International application No.

**PCT/CN2022/139328**

| Patent document cited in search report | | | Publication date (day/month/year) | Patent family member(s) | | | Publication date (day/month/year) |
|---|---|---|---|---|---|---|---|
| CN | 113197671 | A | 03 August 2021 | None | | | |
| CN | 104602619 | A | 06 May 2015 | WO | 2014004255 | A1 | 03 January 2014 |
| | | | | EP | 2866680 | A1 | 06 May 2015 |
| | | | | EP | 2866680 | B1 | 19 August 2020 |
| | | | | US | 2014000411 | A1 | 02 January 2014 |
| | | | | US | 9072536 | B2 | 07 July 2015 |
| | | | | RU | 2015102601 | A | 20 August 2016 |
| | | | | RU | 2647778 | C2 | 19 March 2018 |
| | | | | BR | 112014032653 | A2 | 27 June 2017 |
| | | | | BR | 112014032653 | B1 | 17 May 2022 |
| | | | | JP | 2015521904 | A | 03 August 2015 |
| | | | | JP | 6117354 | B2 | 19 April 2017 |
| CN | 113272104 | A | 17 August 2021 | US | 2022125529 | A1 | 28 April 2022 |
| | | | | WO | 2020167906 | A1 | 20 August 2020 |
| | | | | EP | 3924151 | A1 | 22 December 2021 |
| US | 2020275928 | A1 | 03 September 2020 | BR | 112014032776 | A2 | 27 June 2017 |
| | | | | BR | 112014032776 | B1 | 08 September 2021 |
| | | | | US | 11464513 | B2 | 11 October 2022 |
| WO | 2016026519 | A1 | 25 February 2016 | None | | | |
| CN | 112087983 | A | 15 December 2020 | US | 2021113282 | A1 | 22 April 2021 |
| | | | | EP | 3790490 | A1 | 17 March 2021 |
| | | | | WO | 2019217353 | A1 | 14 November 2019 |

Form PCT/ISA/210 (patent family annex) (July 2022)

**EP 4 450 015 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111541632